(19) **Europäisches Patentamt / European Patent Office / Office européen des brevets**

(11) **EP 2 035 548 B1**

(12) # EUROPEAN PATENT SPECIFICATION

(45) Date of publication and mention of the grant of the patent:
**11.08.2010 Bulletin 2010/32**

(51) Int Cl.:
**G01N 33/68** [(2006.01)]

(21) Application number: **07722668.6**

(22) Date of filing: **15.06.2007**

(86) International application number:
**PCT/DK2007/000291**

(87) International publication number:
**WO 2007/144001 (21.12.2007 Gazette 2007/51)**

(54) **EMBRYO QUALITY ASSESSMENT BASED ON BLASTOMERE DIVISION AND MOVEMENT**

EMBRYONENQUALITÄTSBEURTEILUNG AUF DER GRUNDLAGE VON BLASTOMERENTEILUNG UND BEWEGUNG

ÉVALUATION DE LA QUALITÉ D'UN EMBRYO SUR LA BASE DE LA DIVISION ET DU MOUVEMENT DES BLASTOMÈRES

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **16.06.2006 DK 200600821**
**16.06.2006 US 814115 P**
**16.10.2006 PCT/DK2006/000581**
**19.04.2007 DK 200700571**

(43) Date of publication of application:
**18.03.2009 Bulletin 2009/12**

(73) Proprietor: **UNISENSE FERTILITECH A/S**
**8200 Åarhus N (DK)**

(72) Inventors:
- **RAMSING, Niels B.**
  **8240 Risskov (DK)**
- **BERNTSEN, Jørgen**
  **8800 Viborg (DK)**

(74) Representative: **Høiberg A/S**
**St. Kongensgade 59 A**
**1264 Copenhagen K (DK)**

(56) References cited:
**US-A1- 2003 138 942**

- **HOLM P ET AL: "Development kinetics of the first cell cycles of bovine in vitro produced embryos in relation to their in vitro viability and sex" THERIOGENOLOGY, vol. 50, no. 8, December 1998 (1998-12), pages 1285-1299, XP002455977 ISSN: 0093-691X cited in the application**

- **LEQUARRE A S ET AL: "Cell cycle duration at the time of maternal zygotic transition for in vitro produced bovine embryos: Effect of oxygen tension and transcription inhibition." BIOLOGY OF REPRODUCTION, vol. 69, no. 5, November 2003 (2003-11), pages 1707-1713, XP002455978 ISSN: 0006-3363**

- **GRISART B ET AL: "CINEMATOGRAPHIC ANALYSIS OF BOVINE EMBRYO DEVELOPMENT IN SERUM-FREE OVIDUCT-CONDITIONED MEDIUM" JOURNAL OF REPRODUCTION AND FERTILITY, JOURNALS OF REPRODUCTION AND FERTILITY LTD, GB, vol. 101, no. 2, 1994, pages 257-264, XP009079440 ISSN: 0022-4251 cited in the application**

- **GONZALES D S ET AL: "Prediction of the developmental potential of hamster embryos in vitro by precise timing of the third cell cycle" JOURNAL OF REPRODUCTION AND FERTILITY, JOURNALS OF REPRODUCTION AND FERTILITY LTD, GB, vol. 105, no. 1, September 1995 (1995-09), pages 1-8, XP009090408 ISSN: 0022-4251**

- **TOKURA T ET AL: "Sequential observation of mitochondrial distribution in mouse oocytes and embryos" JOURNAL OF ASSISTED REPRODUCTION AND GENETICS, PLENUM PUBLISHING, US, vol. 10, no. 6, August 1993 (1993-08), pages 417-426, XP009091192 ISSN: 1058-0468**

**EP 2 035 548 B1**

**(Cont. next page)**

- SQUIRRELL J M ET AL: "Imaging mitochondrial organization in living primate oocytes and embryos using multiphoton microscopy" MICROSCOPY AND MICROANALYSIS, SPRINGER, NEW YORK, NY, US, vol. 9, no. 3, June 2003 (2003-06), pages 190-201, XP009091191 ISSN: 1431-9276
- SCHATTEN ET AL: "The significance of mitochondria for embryo development in cloned farm animals" MITOCHONDRION, ELSEVIER, AMSTERDAM, NL, vol. 5, no. 5, October 2005 (2005-10), pages 303-321, XP005088701 ISSN: 1567-7249
- SQUIRRELL J M ET AL: "LONG-TERM TWO-PHOTON FLUORESCENCE IMAGING OF MAMMALIAN EMBRYOS WITHOUT COMPROMISING VIABILITY" NATURE BIOTECHNOLOGY, NATURE PUBLISHING GROUP, NEW YORK, NY, US, vol. 11, no. 17, August 1999 (1999-08), pages 763-767, XP001074651 ISSN: 1087-0156
- VAN BLERKOM J ET AL: "A microscopic and biochemical study of fragmentation phenotypes in stage-appropriate human embryos." HUMAN REPRODUCTION (OXFORD, ENGLAND) APR 2001, vol. 16, no. 4, April 2001 (2001-04), pages 719-729, XP002468388 ISSN: 0268-1161

**Description**

The present invention relates to a method for selecting embryos for in vitro fertilization based on the timing, and extent of observed cell divisions and associated cellular movement.

**Background**

Infertility affects more than 80 million people worldwide. It is estimated that 10% of all couples experience primary or secondary infertility (Vayena et al. 2001). In vitro fertilization (IVF) is an elective medical treatment that may provide a couple who has been otherwise unable to conceive a chance to establish a pregnancy. It Is a process in which eggs (oocytes) are taken from a woman's ovaries and then fertilized with sperm in the laboratory. The embryos created in this process are then placed into the uterus for potential implantation. To avoid multiple pregnancies and multiple births only a few embryos are transferred (normally less than four and ideally only one (Bhattacharya et al. 2004)). Selecting proper embryos for transfer is a critical step in any IVF-treatment. Current selection procedures are mostly entirely based on morphological evaluation of the embryo at different timepoints during development and particularly an evaluation at the time of transfer using a standard stereomicroscope. However, it is widely recognized that the evaluation procedure needs qualitative as well as quantitative improvements.

*Early cell division.* A promising new approach is to use 'early division' to the 2-cell stage, (i.e. before 25-27 h post insemination/injecton), as a quality indicator. In this approach the embryos are visually inspected 25 - 27 hours after fertilization to determine if the first cell division has been completed. Several studies have demonstrated strong correlation between early cleavage and subsequent development potential of individual embryos. (Shoukir et al., 1997; Sakkas et al., 1998, 2001; Bos-Mikich et al., 2001; Lundin et al., 2001; Petersen et al., 2001; Fenwick et al., 2002; Neuber et al. 2003; Salumets et al., 2003; Windt et al., 2004). The need for more frequent observation has been pointed out by several observers. However, frequent visual observations with associated transfers from the incubator to an inverted microscope induce a physical stress that may impede or even stall embryo development. It is also time consuming and difficult to incorporate in the daily routine of IVF clinics.

Several researchers have performed time-lapse image acquisition during embryo development. This has mainly been done by placing a research microscope inside an incubator or building an "incubator stage" onto a microscope stage with automated image acquisition. The "incubator" maintain acceptable temperature (37 °C), humidity (> 90%) and gas composition (5% $CO_2$ and in some cases reduced oxygen concentration). Manual assessment of time-lapse images has yielded important information about timing and time interval between onset of consecutive cell divisions (Grisart et al. 1994, Holm et al. 1998, Majerus et al. 2000, Holm et al. 2002, Holm et al. 2003, Lequarre et al. 2003, Motosugi et al. 2005).

Tokura et al. (1993) discloses that in mouse embryos cultured in vitro showing a developmental block mitochondrial translocation was inhibited after aggregation in the early two-cell stage

Squirrell et al. (2003) reports that distinct accumulation of mitochondria between the pronuclei in fertilised eggs prior to syngamy is positively correlated with development to blastocyst stage in primate embryos.

In Squirrell et al. (1999) mitochondria were tracked in live embryos to the blastocyst stages using multiphoton microscopy.

**Summary of the invention**

The present invention relates to a method to facilitate the selection of optimal embryos to be implanted after in vitro fertilization (IVF) based on the timing, duration, spatial distribution, and extent of observed cell divisions and associated cellular and organelle movement.

Accordingly, in a first aspect the invention relates to a method for determining embryo quality comprising monitoring the embryo for a time period before the embryo undergoes compaction, said time period having a length sufficient to comprise at least one cell division period and at least a part of an inter-division period, and determining the extent of cellular movement during the at least part of an inter-division period thereby obtaining an embryo quality measure.

The obtained embryo quality measure may then be used for identifying and selecting embryos suitable of transplantation into the uterus of a female in order to provide a pregnancy and live-born baby.

Thus, in a further aspect the invention relates to a method for selecting an embryo suitable for transplantation, said method comprising monitoring the embryo as defined above obtaining an embryo quality measure, and selecting the embryo having the highest embryo quality measure.

The invention may be applied in a system having means for carrying out the methods described above. Said system may be any suitable system, such as a computer comprising computer code portions constituting means for executing the methods as described above. The system may further comprise means for acquiring images of the embryo at different time intervals, such as the system described in WO2007/042044.

The invention may be applied in a data carrier comprising computer code portions constituting means for executing the methods as described above.

**Drawings**

Fig. 2 Blastomere activity of two representative bovine embryos "Good" developed to a hatching bastocyst. "Bad" never developed to blastocyst.

Fig. 3 Blastomere activity of 41 bovine embryos. The blastomere activity is displayed as a pseudo-gel-image where motility peaks are indicated by dark bands and inactivity is white each lane corresponds to a single embryo. The dark banding pattern or smears reflect periods of cellular motility within the embryo. "Good" embryos developing to blastocysts shown above "bad" embryos that did not develop to the blastocyst stage. More sharp Initial bands (usually three) are seen for good embryos.

Fig. 4 Blastomere activity of thirteen representative bovine embryos. "Good" embryos developed to a hatching bastocyst are represented by green curves. "Bad" embryos never developed to blastocyst are shown in read. X-axis is frame number y-axis is blastomere activity. Image acquisition started 24 hours after fertilization and progressed with 2 frames per hour. The green curves have been displaced on the y-axis by adding 30 to the blastomere activity value.

Fig. 5 Average blastomere activity for all acquired frames (Light area = high blastomere activity, dark area = low blastomere activity).

Fig. 6 Blastomere activity of 21 bovine embryos that did not develop to high quality blastocysts. The three parts of the curves that are used to classify the blastomere activity pattern are indicated.

Fig.7 Blastomere activity of 18 bovine embryos that did not develop to high quality blastocysts. The three parts of the curves that are used to classify the blastomere activity pattern are indicated.

Fig. 8 Corellation between cell divisions detected manually and automatically for 13 representative embryos. About 10 % of the cell divisions were not detected by this algorithm, but otherwise the correspondence is excellent.

Fig. 9. Manually detected cell divisions for good and bad embryos.

Fig. 10. Estimation of derived parameters. The graph in the upper right corner shows the original blastomere activities as a function of frame number. The green and blue line indicates the start of second and third time interval, respectively. The graph in the lower right corner shows the derived parameters, as described above. The vertical red lines indicate the time and value of the highest or lowest activity values within a peak or valley, respectively.

Fig. 11. Derived parameters (see figure above) from blastomer activity analysis of 94. embryos. The embryos that develop to good quality expanded blast are shown in red (good examples) the ones that do not are shown in blue (bad examples).

Fig.12. PCA plot of the first five PCA axes. A red point is an embryo with good quality while blue is an embryo with poor quality.

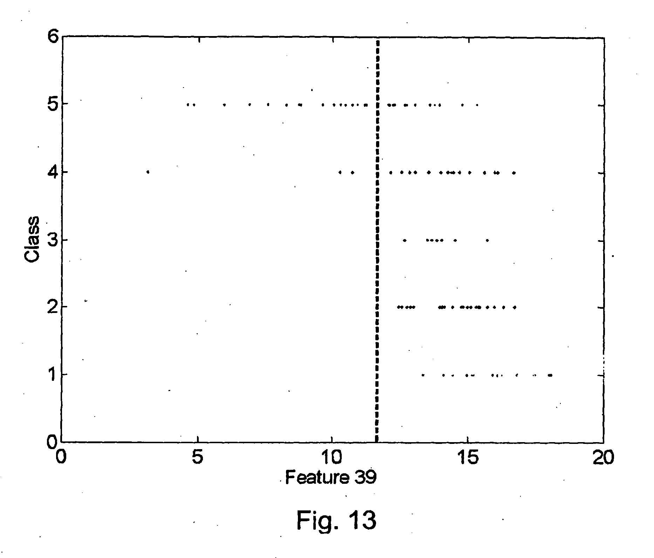

Fig. 13 Baseline value for blastomere activity in time segment 3 (i.e. 76 to 96 hours after fertilization) for 94 different embryos. The grade is a measure of the blastomere quality of the given bovine embryo after 7 days of incubation. Grade 1 embryos are the best quality and have significantly higher baseline values than grade 5 which are the lowest quality and often attretic.

**Detailed description of the invention**

**Definitions**

Cell division period: the period of time from the first observation of indentations In the cell membrane (indicating onset of cytoplasmic division) to the cytoplasmic cell division is complete so that the cytoplasm of the ensuing daughter cells is segregated in two separate cells.

Inter-division period: the period of time from end of one cell division period to the onset of the subsequent cell division period.

Division cycle: The time interval between onset of consecutive cell divisions i.e. from start of one cell division period to start of the subsequent cell division

## Rearrangement of cellular position = Cellular movement (see below)

Cellular movement: Movement of the center of the cell and the outer cell membrane. Internal movement of organelles within the cell is NOT cellular movement. The outer cell membrane is a dynamic structure, so the cell boundary will continually change position slightly. However, these slight fluctuations are not considered cellular movement. Cellular movement is when the center of gravity for the cell and its position with respect to other cells change as well as when cells divide. Cellular movement can be quantified by calculating the difference between two consecutive digital images of the moving cell. An example of such quantification is described in detail in WO 2007/042044. However, other methods to determine movement of the cellular center of gravity, and or position of the cytoplasm membrane may be envisioned e.g. by using FertiMorph software (ImageHouse Medical, Copenhagen, Denmark) to semiautomatically outline the boundary of each blastomere in consecutive optical transects through an embryo.

Organelle movement: Movement of internal organelles and organelle membranes within the embryo which may be visible by microscopy. Organelle movement is not Cellular movement in the context of this application.

Movement: spatial rearrangement of objects. Movements are characterized and/or quantified and/or described by many different parameters including but restricted to: extent of movement, area and/or volume involved in movement, rotation, translation vectors, orientation of movement, speed of movement, resizing, inflation/deflation etc. Different measurements of cellular or organelle movement may thus be used for different purposes some of these reflect the extent or magnitude of movement, some the spatial distribution of moving objects, some the trajectories or volumes being afflicted by the movement.

Embryo: In some cases the term "embryo" is used to describe a fertilized oocyte after implantation in the uterus until 8 weeks after fertilization at which stage it becomes a foetus. According to this definition the the fertilized oocyte is often called a pre-embryo until implantation occurs. However, throughout this patent application we will use a broader definition of the term embryo, which includes the pre-embryo phase. It thus encompasses all developmental stages from the fertilization of the oocyte through morula, blastocyst stages hatching and implantation.

Embryo quality is a measure of the ability of said embryo to successfully implant and develop in the uterus after transfer. Embryos of high quality will successfully implant and develop in the uterus after transfer whereas low quality embryos will not.

Embryo viability is a measure of the ability of said embryo to successfully implant and develop in the uterus after transfer. Embryos of high viability will successfully implant and develop in the uterus after transfer whereas low viability embryos will not. Viability and quality are used interchangeably in this document

Embryo quality (or viability) measurement is a parameter intended to reflect the quality (or viability) of an embryo such that embryos with high values of the quality parameter have a high probability of being of high quality (or viability), and low probability of being low quality (or viability). Whereas embryos with an associated low value for the quality (or viability) parameter only have a low probability of having a high quality (or viability) and a high probability of being low quality (or viability)

## Embryo

An embryo is approximately spherical and is composed of one or more cells (blastomeres) surrounded by a gelatine-like shell, the acellular matrix known as the zona pellucida. The zona pellucida performs a variety of functions until the embryo hatches, and is a good landmark for embryo evaluation. The zona is spherical and translucent, and should be clearly distinguishable from cellular debris.

An embryo is formed when an oocyte is fertilized by fusion or injection of a sperm cell (spermatozoa). The term is traditionally used also after hatching (i.e. rupture of zona pelucida) and the ensuing implantation. For humans the fertilized oocyte is traditionally called an embryo for the first 8 weeks. After that (i.e. after eight weeks and when all major organs have been formed) it is called a foetus. However the distinction between embryo and foetus is not generally well defined.

During embryonic development, blastomere numbers increase geometrically (1-2-4-8-16- etc.). Synchronous cell division is generally maintained to the 16-cell stage in embryos. After that, cell division becomes asynchronous and finally individual cells possess their own cell cycle. For bovine embryos: The blastomeres composing the embryo should be easily identifiable until at least the 16-cell stages as spherical cells. At about the 32-cell stage (morula stage), embryos undergo compaction, as inter-cell adhesion occur when adhesion proteins are expressed. As a result, individual cells in the embryo are difficult to evaluate an enumerate beyond this stage. For human embryos compaction occurs somewhat earlier and individual blastomeres can not readily be identified at the 16 cell stage. Human embryos produced during infertility treatment are usually transferred to the recipient before the morula stage, whereas other mammalian embryos often are cultured experimentally to a further development stage (expanded blastocysts) before transfer to the recipient or discharge. In some cases human embryos are also cultivated to the blastocyst stage before transfer. This is preferably done when many good quality embryos are available or prolonged incubation is necessary to await the result of a preimplantation genetic diagnosis (PGD). Accordingly, the term embryo is used in the following to denote each of the stages fertilized oocyte, zygote, 2-cell, 4-cell, 8-cell, 16-cell, morula, blastocyst, expanded blastocyst and hatched blastocyst, as well as all stages in between (e.g. 3 - cell or 5-cell)

**Determination of quality**

The present invention provides an embryo quality measurement [See definition of embryo quality measurement above] being based on one or more determinations of the embryo, such as determining the extent of cellular movement in at least two inter-division periods thereby obtaining an embryo quality measure.

The invention relies on the observation that the cell positions are usually relatively stationary between cell divisions (i.e. little cellular movement), except for a short time interval around each cell division, where the division of one cell into two leads to brief but considerable rearrangement of the dividing cells as well as the surrounding cells (i.e. pronounced cellular movement).

A particular use contemplated by this application is to evaluate image series of developing embryos (time-lapse images). These time-lapse images may be analyzed by difference imaging (see WO 2007/042044). The resulting difference images can be used to quantify the amount of change occurring between consecutive frames in an image series.

The application may be applied to analysis of difference Image data, where the changing positions of the cell boundaries (i.e. cell membranes) as a consequence of cellular movement causes a range parameters derived from the difference image to rise temporarily (see WO 2007/042044). These parameters include (but are not restricted to) a rise in the mean absolute intensity or variance. Cell divisions and their duration and related cellular re-arrangement can thus be detected by temporary change, an increase or a decrease, in standard deviation for all pixels in the difference image or any other of the derived parameters for "blastomere activity" listed in WO 2007/042044. However the selection criteria may also be applied to visual observations and analysis of time-lapse images and other temporally resolved data (e.g. excretion or uptake of metabolites, changes in physical or chemical appearance, diffraction, scatter, absorption etc.) related to embryo development that are not related to blastomere activity as defined in WO 2007/042044.

Of particular interest are the onset, magnitude and duration of cell divisions that may be quantified as peaks or valleys; in derived parameter values. These extremes, peaks or valleys, frequently denote cell division events The timing and duration of these events as well as the parameter values observed during and between the events are used to characterize the embryo, and to evaluate its development potential. The shape of each peak also provides additional information as may the size of the peak in general. A peak may also denote an abrupt collapse of a blastomer and concurrent cell death. However, it may be possible to separate cell division events and cell death events by the peak shape and change in base values before and after the event. The baseline of most parameters are usually not affected by cell division whereas cell lysis is frequently accompanied by a marked change in the baseline value (for most parameters in a decrease following lysis.)

Another particular interest is the spatial distribution of both cellular and organelle movement. Volumes within the zona pelucida that are devoid of movement (or similarly areas in a projected 2D image of the embryo that remain stationary) are an indication of "dead" zones within the embryo. The more and larger these immotile "dead" zones the lower the probability of successful embryo development. Large areas within a time-lapse series of embryo images without any type of movement (i.e. neither cellular nor organelle movement) indicates low viability. Organelle movement should generally be detectable in the entire embryo even when only comparing two or a few consecutive frames. Cellular movement may be more localized especially in the later phases of embryo development, However, when evaluating many successive frames cellular movement should be detectable in the entire volume within the Zona Pelucida, which indicates that all blastomeres within the embryo divide and change position. Thus, the embryo quality measure according to application comprises information about cellular and organelle movement during at least one cell division, and/or at least a part of one inter-division period such as i) the duration of the at least one cell division period; and/or ii) determining the extent and/or spatial distribution of cellular or organelle movement during the cell division period; and/or iii) determining duration of an inter-division period; and/or iv) determining the extent and/or spatial distribution of cellular or organelle movement during the inter-division period. The embryo quality measure may comprise information of two or more of the determinations described herein, such as three or more of the determinations described herein. The embryo quality measure may comprise information of all the determinations described herein. In particular the embryo quality measure comprises information about the length of the cell division period and the length of the interdivision period, or the embryo quality measure comprises information comprises information about the movement in the cell division period and the movement in the interdivision period. The embryo quality measure may comprise information about the length of a period and the movement in the same period.

The embryo quality measure is based on the following observations:

a) Abrupt cell divisions where the actual division of the cytoplasm proceeds rapidly and the ensuing re-arrangement of the positions of the other blastomeres occur rapidly (e.g. sharp blastomere activity peaks) is indicative of a high quality embryo. Prolonged duration of cytoplasmic division and extensive spatial rearrangement of the other blastomeres afterwards (i.e. cellular movement) indicate a poor quality embryo (e.g. broad blastomere activity peaks). (Example 1)

b) Little rearrangement of blastomere position between cell divisions indicates a high quality embryo whereas movement between visible cell divisions often indicates a poor quality embryo. (Example 1)

c) Prolonged rearrangement of cell position between cell division (e.g. broad blastomere activity peaks) is often associated with poor embryo quality, asynchronous cell division and extensive fragmentation. (Example 1)

d) A quiet period of very little cellular movement is observed for most mammals when the embryonic genome is activated and protein synthesis switches from maternal to embryonal transcripts. If this period has: i) Early onset, ii) very low activity (= little cellular movement = quiet) and iii) early termination then it is a strong indication of a high quality embryo. The onset of the quiet period is often delayed, and the period is sometimes interrupted by cellular movement in poor quality embryos. Poor quality embryos may also have an elevated baseline level of cellular movement in the "quiet" period without detectable cell division (Example 2)

e) In poor quality embryos that subsequently cease development particular and persistently immobile regions are often observed which persist and ultimately lead to developmental arrest. Such immobile regions may be associated with extensive fragmentation or blastomere death and lysis. If these regions are larger than a given percentage at a given developmental stage then the embryo has very low probability to survive. In high quality embryos the cellular motility that ensue briefly after each cytoplasmic division event is initially distributed over the entire embryo surface (i.e. all blastomeres move slightly), only after compaction in the morula stage is localized movement seen (Example 3).

f) A uniform spatial distribution of organelle movement is generally found in viable high quality embryos, whereas "Dead" zones devoid of motility are frequently found for low quality embryos. Similar observations have been made for cellular movement, but observation during a longer time-window is required to determine the spatial uniformity of the cellular movement (Example 3).

g) The amount of cellular movement in different time intervals is a good indicator of embryo quality. A quality related parameter can be calculated from a ratio of average movement in different time-segments and/or a ratio of standard deviations in different time-segments Embryo selection procedures can be established based on the value of these parameters. (Example 4).

h) A gradual or abrupt decrease in the baseline level of cellular motility and organelle motility is frequently associated with low embryo quality and a high probability of developmental arrest. The change in baseline level may be associated with emergence of inactive zones/regions (see (e) and (f) above). (example 6)

i) Early onset of the first cell division is an indication of high embryo quality. Late onset of first (and subsequent cell divisions) indicates low quality embryos. However, for the majority of the embryos, the exact onset of the first cell division alone does not provide a clear indication of embryo quality (Example 4)

j) For most of the derived parameters describing cellular and organelle movement a normal range can be defined such that values outside the normal range (e.g. abnormally high or abnormally low) are both indicative of poor embryo quality. (Example 6)

k) The intervals between consecutive cell divisions are important (and species specific) indicators of embryo viability an example would be the ration between the interval between $1 \rightarrow 2$ and $2 \rightarrow 4$ cell division and the interval between the $2 \rightarrow 4$ and the $4 \rightarrow 8$ cell division. The ration of these intervals should be within a given range for viable embryos.

l) Synchronized cell division in the later stages (e.g. 2→4, 4→8) is mostly found for high quality embryos whereas asynchronous cell division is often observed for low quality embryos (e.g. 2→3→4→5→6→7→8) (Example 1)

The following determinations lead to the highest embryo quality measure:

- Short cell-division periods, wherein short is defined as less than 2 hour
- Little cellular movement In inter-dlvision periods, wherein little is defined as virtually no change in cellular position beyond the usual oscillations and organelle movements that always contribute to the difference image. Little cellular movement imply that the cellular center of gravity is stationary (movement < 3 $\mu$m) and the cytoplasmic membranes are largely immotile (< 3 $\mu$m).
- Early onset of first cell-division period, i.e. before 25 hours after fertilisation for human embryos (before 30 hours after fertilisation for bovine embryos).
- Short periods of cellular movements in inter-division periods, wherein short is defined as less than 3 hours
- Uniform distribution of cellular movement within the Zona pelucida over time, i.e. absence of inactive areas/zones/volumes of the embryo where cellular movement is not observed over a longer period of time (i.e. > 24 hours). Such immobile zones could be due to dead or dying blastomeres and fragments, which may impede further development
- Constant or slightly increasing baseline values for cellular motility
- All derived parameters were within the normal range for the particular embryo

The closer the embryo quality measure gets to the highest quality measure the higher quality for the embryo.
A neural network or other quantitative pattern recognition algorithms may be used to evaluate the complex cell motility patterns described above. Such a network may be used to find the best quality embryos for transfer in IVF treatments. Example 6 describes an approach to derive key parameters for embryo development from "Blastomere activity" (see WO 2007/042044) during embryo development, and subsequently evaluate the derived parameters using different mathematical models (linear, Princepal component analysis, Markov models etc.)

## Other measurements

A final analysis step could include a comparison of the made observations with similar observations of embryos of different quality and development competence, as well as comparing parameter values for a given embryo with other quantitative measurements made on the same embryo. This may include a comparison with online measurements such as blastomer motility, respiration rate, amino acid uptake etc. A combined dataset of blastomer motility analysis, respiration rates and other quantitative parameters are likely to improve embryo selection and reliably enable embryologist to choose the best embryos for transfer.
The method may be combined with other measurements in order to evaluate the embryo in question, and may be used for selection of competent embryos for transfer to the recipient.
Such other measurements may be selected from the group of respiration rate, amino acid uptake, motility analysis, blastomer motility, morphology, blastomere size, blastomere granulation, fragmentation, blastomere color, polar body orientation, nucleation, spindle formation and integrity, and numerous other qualitative measurements. The respiration measurement may be conducted as described in PCT publication no. WO 2004/056265.

## Culture medium

The observations may be conducted during cultivation of the cell population, such as wherein the cell population is positioned in a culture medium. Means for culturing cell population are known in the art. An example of culturing an embryo is described in PCT publication no. WO 2004/056265.

## Data carrier

The application further relates to a data carrier comprising a computer program directly loadable in the memory of a digital processing device and comprising computer code portions constituting means for executing the method of the invention as described above.
The data carrier may be a magnetic or optical disk or in the shape of an electronic card of the type EEPROM or Flash, and designed to be loaded into existing digital processing means.

Selection or identification of embryos

The present invention further provides a method for selecting an embryo for transplantation. The method implies that the embryo has been monitored for determining a change in the embryo as described above in order to determine when cell divisions have occurred and optionally whether cell death has occurred as well as the quality of cell divisions and overall quality of embryo. It is preferred to select an embryo having substantially synchronous cell division giving rise to sharp derived parameters for the difference images, and more preferred to select an embryo having no cell death.

The selection or identifying method may be combined with other measurements as described above in order to evaluate the quality of the embryo. The important criteria in a morphological evaluation of embryos are: (1) shape of the embryo including number of blastomers and degree of fragmentation; (2) presence and quality of a zona pellucida; (3) size; (4) colour and texture; (5) knowledge of the age of the embryo in relation to its developmental stage, and (6) blastomere membrane integrity.

The transplantation may then be conducted by any suitable method known to the skilled person.

**Example 1**

*Materials and methods.* Bovine immature cumulus-oocyte complexes (COCs) were aspirated from slaughterhouse-derived ovaries, selected and matured for 24 h in four-well dishes (Nunc, Roskilde, Denmark). Each well contained 400 $\mu$L of bicarbonate buffered TCM-199 medium (Gibco BRL, Paisley, UK) supplemented with 15% cattle serum (CS; Danish Veterinary Institute, Frederiksberg, Denmark), 10 IU/mL eCG and 5 IU/mL hCG (Suigonan Vet; Intervet Scandinavia, Skovlunde, Denmark), The embryos were matured under mineral oil at 38.5 °C in 5% CO2 in humidified air. Fertilization was performed in modified Tyrode's medium using frozen-thawed, Percoll-selected sperm. After 22 h, cumulus cells were removed by vortexing and presumptive zygotes were transferred to 400 $\mu$L of culture medium, composed of synthetic oviduct fluid medium with aminoacids, citrate and inositol (SOFaaci) supplemented with antibiotics (Gentamycin sulfate, 10mg/ml) and 5% CS and incubated at 38.5 °C in 5% CO2, 5% 02, 90% N2 atmosphere with maximum humidity.

The incubator system has been described in detail earlier and has proved suitable for in-vitro embryo culture (Holm et al. 1998). Briefly, the 4-well culture dish was placed on the microscopic stage (MultiControl 2000 Scanning stage, Märzhäuser, Germany) of an inverted Nikon TMD microscope (Diaphot, DFA A/S, Copenhagen, Denmark). A black plexiglas incubator box regulated by an air temperature controller (Air-Therm™, World Precision Instruments, Aston, UK) was fitted around the stage. A plastic cover with open bottom was placed over the culture dish and the humidified gas-mixture was lead into this semi-closed culture chamber after having passed through a gas washing bottle placed inside the incubator box.

This culture box has previously been proved useful for in-vitro embryo culture (Holm et al. 1998, 2003), providing stable temperature and humidity conditions. Our weekly routine in vitro embryo production during the experimental served as controls for the integrity of the basic culture system.

Camera system. The time-lapse recording was directed by an image analysis software (ImagePro™, Unit One, Birkerød, Denmark), which controlled both the movements of the scanning stage in the x-, y- and z-axes, the operation of the connected highly light sensitive video camera (WAT-902H, Watec, DFA A/S, Copenhagen, Denmark), as well as the recording and storage of time-lapse sequences on the computer hard disc. Time-lapse Images of each embryo (total magnification: x 265) were sequentially recorded in minimal light at intervals of 30 min. throughout the 7 day culture period. Between recordings the embryo were moved out of the light field.

Manual analysis of the time-lapse image series consisted of recording the time of the first appearance of the following cleavage/embryo stages: 2-cell, 4-cell, 8-cell, 16-cell and for morulae and blastocysts with a visible coherent cell mass: maximal compact morula, first expansion of the blastocyst, collapses of blastocysts and hatching of the blastocyst.

The automated computer based analysis consisted of computing the standard deviation of the differences image which is calculated as the difference between two consecutive frames. To avoid alignment artifacts and other problems the following elaborate procedure was used:

    1) *Image acquisition.* (See description above).

    2) *Remove fixed position artifacts (Camera dust)* by subtracting a defocused reference image of the artifacts from every picture in the series.

    3) *Translocation to compensate for inaccurate* stage *movement.* A very simple way to align pictures is to compare the original difference image to a difference image calculated after shifting one of the original images a single pixel in a given direction. If the variance of the difference image calculated after translocation is lower than the variance of the difference image of the originals then the translocation produced an improved alignment. By systematically trying out all possible translocation directions and all relevant translocation magnitudes it is possible to obtain an aligned time series. However in the present case we used an advanced ImageJ macro for image alignment developed by Thévenaz et al. 1998.

    4) *Identify region of interest (ROI) and reference area outside.* It is advantageous to compare cell movement inside the embryo to "movement" outside the embryo due Brownian motion alignment problems etc. This is accomplished by delineating the embryo and comparing the difference images inside the embryo witch the calculated differences in a similar area outside the embryo. Delineating the embryo was done manually. A reference area we chose a region of the image without any embryos.

*5) Calculate intensity difference.*

*6) Compute a derived parameters for each difference image.* Several difference parameters were calculated but the one that proved most informative was the standard deviation of intensity for all pixels in the difference image. This parameter is refered to as the "blasomere activity" in the following

*7) Identify and determine shape* of *peaks in the blastomere activity.*

*8) Calculate standard deviation and average values for the blastomere activity for diagnostically relevant time intervals* See example 4.

Experimental design. Approx. 20 bovine embryos were incubated together in a single well of a Nunc-4well dish for 7 days with image acquisition every 30 min. This experiment was repeated 5 times total giving time-lapse image series of 99 bovine embryos.

*Results:*

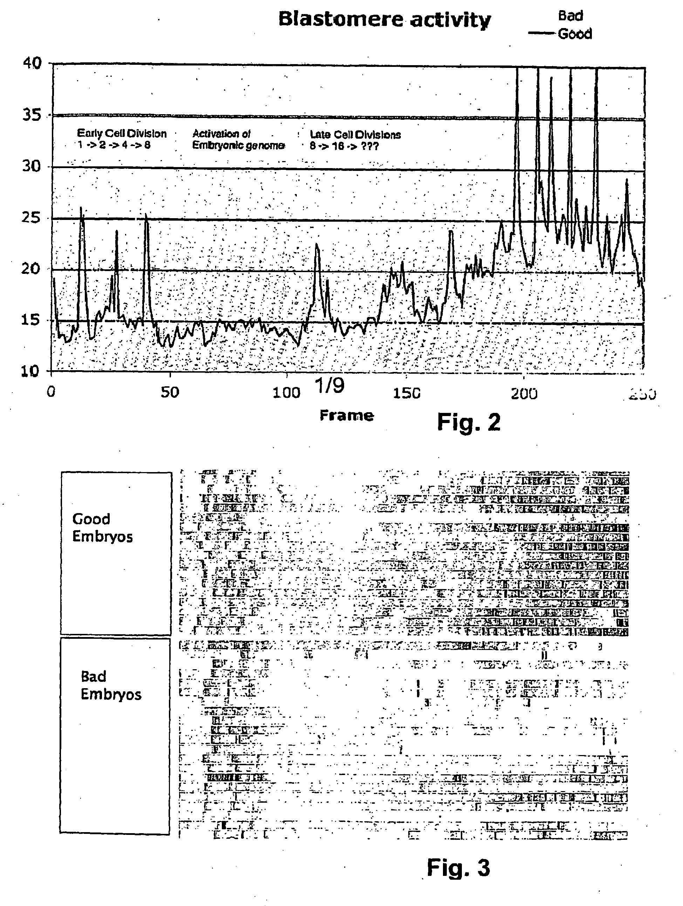

Based on qualitative evaluation of time-lapse image series of developing embryos, (essentially by looking playing them as movies numerous times and noting changes), we observed that: An indicator of high quality embryos is abrupt cell divisions where the actual division of the cytoplasm proceeds rapidly and the ensuing re-arrangement of the positions of the other blastomeres occur rapidly followed by a period of "quiet" with very little rearrangement of cell position until the abrupt onset of the next cytoplasmic division. Poor quality embryos often show prolonged rearrangements of blastomere position after cytoplasmic divisions and between cytoplasmic cell divisions. To quantify and document these observations we calculate blastomere activity from a time-lapse image series as described in WO 2007/042044. Representative blastomere activities are shown in the Fig. 2.

Some of the observed activity is due to asynchronous cell division (e.g. 2→3→4→5→6→7→8) and fragmentation as opposed to synchronous cell divisions (e.g. 2→4, 4→8) observed for high quality embryos.

The blastomere activity of 41 embryos is displayed as a pseudo-gel-image in Fig. 1 where motility peaks are indicated by dark bands and inactivity is white.

**Example 2**

*Materials and methods.* Same as for Example 1

*Results*

Initial protein synthesis in mammalian embryos use maternal mRNA from the oocyte, but after a few cell divisions the embryonic genome is activated, transcribed and translated. The switch from maternal genome to embryonic genome is a crucial step in embryo development. The period occurs at the 8-cell stage for bovines and has a relatively long duration for human embryos the swith occurs earlier at the 4 to 8 cell stage and has a shorter duration.

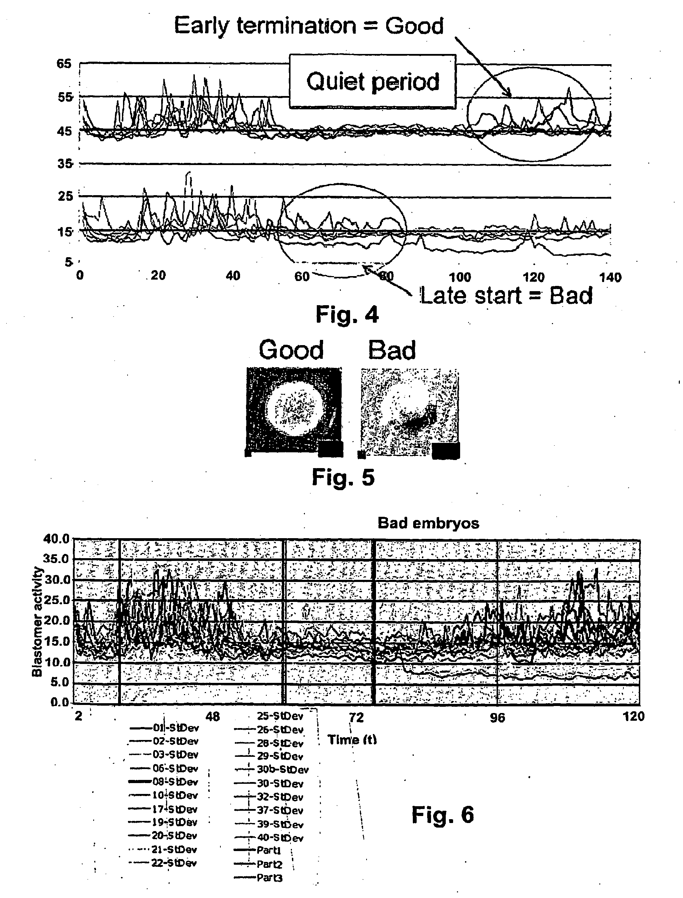

A quiet period of very little cellular movement is observed for most mammals when the embryonic genome is activated and protein synthesis switches from maternal to embryonal genes. If this period has: i) Early onset, ii) very low activity (= little cellular movement = quiet) and iii) early termination then it is a strong indication of a high quality embryo. The quiet period is often delayed, and sometimes interrupted by cellular movement in poor quality embryos. An example of this showing blastomere activity for 13 different embryos is shown in Fig. 4.

**Example 3**

*Materials and methods.* Same as for Example 1

*Results.* In poor quality embryos that subsequently cease development particular and persistently immobile regions are often observed which persist and ultimately lead to developmental arrest. Such immobile regions may be associated with extensive fragmentation or blastomere death and lysis. If these regions are larger than a given percentage at a given developmental stage then the embryo has very low probability to survive. In high quality embryos the cellular motility that ensue briefly after each cytoplasmic division event is initially distributed over the entire embryo surface (i.e. all blastomeres move slightly), only after compaction in the morula stage is localized movement seen

Embryos that develop to blastocysts such as the left panel in Fig. 5 have uniformly distributed blastomere activity. Embryos that do not have uniformly distributed blastomere activity such as the right panel in Fig. 5 never develops into a blastocyst.

**Example 4**

*Materials and methods.* Same as for Example 1

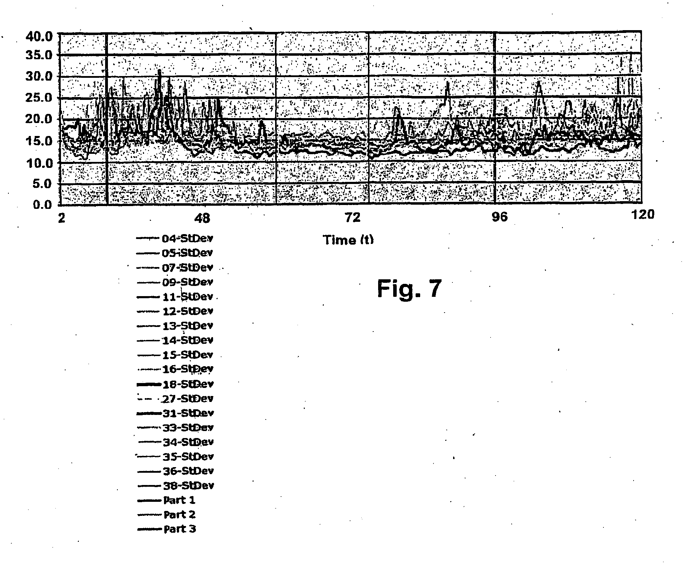

*Results.* The amount of cellular movement in different time intervals is a good indicator of embryo quality. A quality related parameter can be calculated from a ratio of average movement in different time-segments and/or a ratio of standard deviations in different time-segments Embryo selection procedures can be established based on the value of these parameters. Example of different segments (= parts) are shown on Figs. 6 and 7. In this case is part 1 the time segment from 32 to 60 hours after fertilization, part 2 is 60 to 75 hours after fertilization, part 3 is from 75 to 96 hours after fertilization.

Based on the average blastomer activity and/or the standard deviation of the blastomere activity in the different parts it is possible to classify the embryos.

In the present case we have used the following selection criteria based on:

- R1 = ratio between average blastocyst activity in part 1 and in part 3 of the blastocyst activity pattern for a given embryo
- R2 = ration between standard deviation of the blastocyst activity in part 2 and in part 3 of the blastocyst activity pattern for a given embryo

The calculations are shown in Table 1 below

Embryo selection based on ratios of average and of standard deviation for blastomere activity in different segments

| Sections | | Time (h) Frame | | | Time (f Frame | | | Time (f Frame | | |
|---|---|---|---|---|---|---|---|---|---|---|
| | Part 1 | 32 | 73 | Part 2 | 60 | 129 | Part 3 | 75 | 159 | |
| | | 60 | 129 | | 75 | 159 | | 96 | 201 | |

## Bad embryos

| Criteria 1 | | | | | | | | | | | | | | | | | | | | | | |
|---|---|---|---|---|---|---|---|---|---|---|---|---|---|---|---|---|---|---|---|---|---|---|
| | Average | Part 1 | 16.2 | 17.0 | 12.7 | 15.3 | 14.7 | 17.6 | 21.7 | 16.7 | 17.4 | 15.8 | 16.2 | 19.3 | 15.4 | 18.9 | 12.9 | 15.4 | 15.4 | 16.6 | 16.2 | 13.8 | 20.1 |
| | Average | Part 3 | 13.8 | 15.5 | 8.8 | 14.4 | 14.4 | 14.9 | 15.9 | 13.7 | 14.6 | 11.3 | 12.3 | 14.3 | 12.8 | 13.5 | 10.9 | 12.0 | 12.2 | 8.5 | 13.9 | 10.5 | 16.6 |
| | Ratio < | 1.15 | 1.17 | 1.09 | 1.44 | 1.07 | 1.02 | 1.18 | 1.37 | 1.22 | 1.19 | 1.41 | 1.32 | 1.35 | 1.20 | 1.40 | 1.18 | 1.28 | 1.25 | 1.96 | 1.17 | 1.31 | 1.21 |

| Criteria 2 | | | | | | | | | | | | | | | | | | | | | | |
|---|---|---|---|---|---|---|---|---|---|---|---|---|---|---|---|---|---|---|---|---|---|---|
| | StDev | Part 2 | 0.655 | 0.709 | 1.473 | 0.628 | 0.425 | 1.405 | 0.579 | 0.752 | 0.693 | 0.99 | 1.934 | 1.582 | 1.06 | 0.495 | 0.737 | 0.498 | 0.489 | 1.364 | 0.671 | 3.352 | 0.898 |
| | StDev | Part 3 | 3.138 | 0.983 | 1.584 | 1.094 | 1.377 | 1.206 | 2.917 | 1.157 | 0.944 | 1.977 | 1.354 | 2.092 | 1.086 | 0.581 | 0.605 | 1.644 | 1.703 | 2.029 | 2.319 | 4.546 | 1.05 |
| | Ratio < | 0.50 | 0.209 | 0.721 | 0.930 | 0.574 | 0.309 | 1.165 | 0.198 | 0.650 | 0.734 | 0.500 | 1.429 | 0.756 | 0.976 | 0.852 | 1.219 | 0.303 | 0.287 | 0.672 | 0.289 | 0.737 | 0.855 |

Combined

## Good embryos

| Criteria 1 | | | | | | | | | | | | | | | | | | | |
|---|---|---|---|---|---|---|---|---|---|---|---|---|---|---|---|---|---|---|---|
| | Average | Part 1 | 15.6 | 15.2 | 15.3 | 15.9 | 17.4 | 15.8 | 16.0 | 16.1 | 16.0 | 15.6 | 17.1 | 14.7 | 15.6 | 19.7 | 19.7 | 15.8 | 15.2 | 16.6 |
| | Average | Part 3 | 16.3 | 15.9 | 15.8 | 15.9 | 17.3 | 15.1 | 14.8 | 16.6 | 14.0 | 15.6 | 14.0 | 15.0 | 12.1 | 17.9 | 17.8 | 15.3 | 15.0 | 14.8 |
| | Ratio < | 1.15 | 0.96 | 0.95 | 0.97 | 1.00 | 1.01 | 1.05 | 1.08 | 0.97 | 1.14 | 1.00 | 1.22 | 0.98 | 1.29 | 1.10 | 1.11 | 1.03 | 1.02 | 1.12 |

| Criteria 2 | | | | | | | | | | | | | | | | | | | |
|---|---|---|---|---|---|---|---|---|---|---|---|---|---|---|---|---|---|---|---|
| | StDev | Part 2 | 0.909 | 0.519 | 0.444 | 0.524 | 0.632 | 0.75 | 0.518 | 0.581 | 0.699 | 0.703 | 0.92 | 0.79 | 0.413 | 0.888 | 0.909 | 0.551 | 0.504 | 0.473 |
| | StDev | Part 3 | 1.824 | 2.347 | 2.211 | 1.544 | 2.88 | 2.25 | 1.328 | 2.662 | 1.433 | 1.536 | 2.045 | 2.558 | 0.677 | 2.103 | 3.043 | 2.075 | 1.671 | 1.71 |
| | Ratio < | 0.50 | 0.498 | 0.221 | 0.201 | 0.339 | 0.219 | 0.333 | 0.390 | 0.218 | 0.488 | 0.458 | 0.450 | 0.309 | 0.609 | 0.422 | 0.299 | 0.265 | 0.302 | 0.277 |

Combined

*Table 1 Application of selection criteria based on A) R1 = ratio between average blastocyst activity in part 1 and in part 3 of the blastocyst activity pattern. B)R2 = ration between standard deviation of the blastocyst activity in part 2 and in part 3 of the blasocyst activity pattern.*

EP 2 035 548 B1

If (R1 < 1.15 and R2 < 0.50) then it is a "good" embryo ELSE it is a "bad" embryo. Using these criteria all 36 out of 39 embryos were classified correctly according to how they subsequently developed.

## Example 5

*Materials and methods.* Same as for Example 1

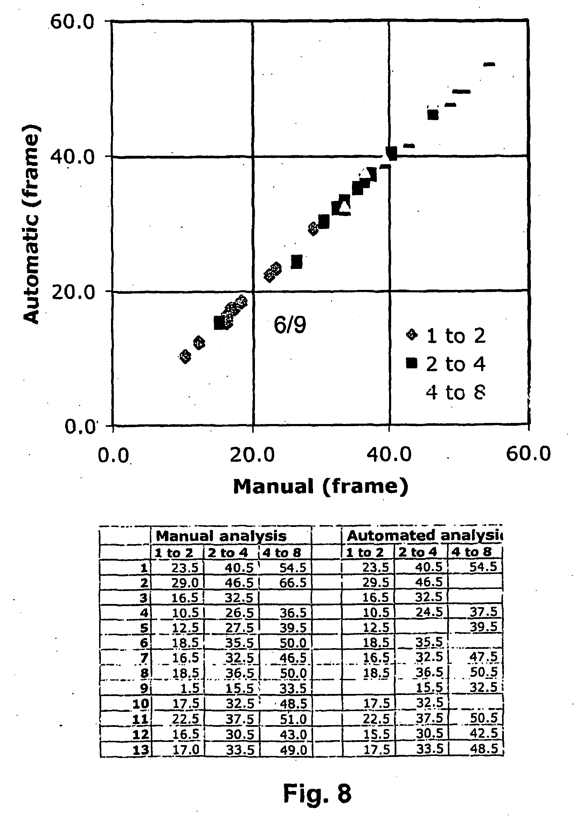

*Results.* Fig. 8 below shows the excellent correspondence between automatic and manual determination of onset of cell division. Very early onset of the first cell division is an indication of high embryo quality. Very late onset of first (and subsequent cell divisions) indicates low quality embryos. However, for the majority of the embryos, the exact onset of the first cell division alone does not provide a clear indication of embryo quality as is shown in Fig. 8 below. While the average onset of cell divisions was delayed for the bad embryos, the large inherent standard deviation makes the absolute values a poor selection criteria except in extreme cases. (e.g. first division before 30 hours signifies a good embryo. First division after 35 hours signifies a bad embryo but the vast majority of the bovine embryos investigated have intermediate division times that are not easily interpreted.

## Example 6a

*Materials and methods.* Same as for Example 1

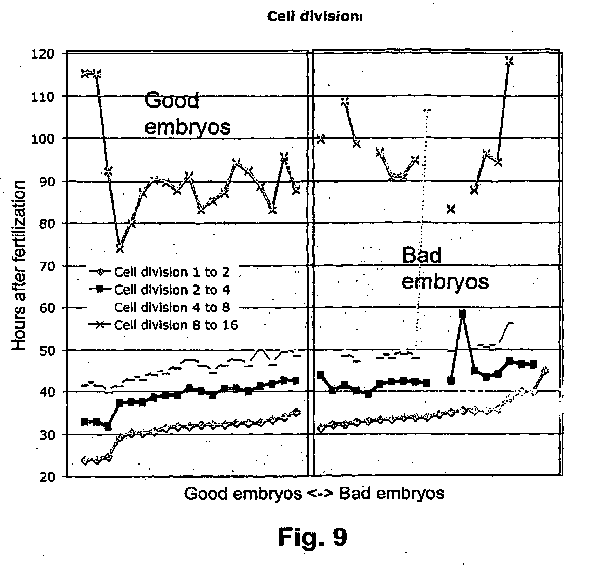

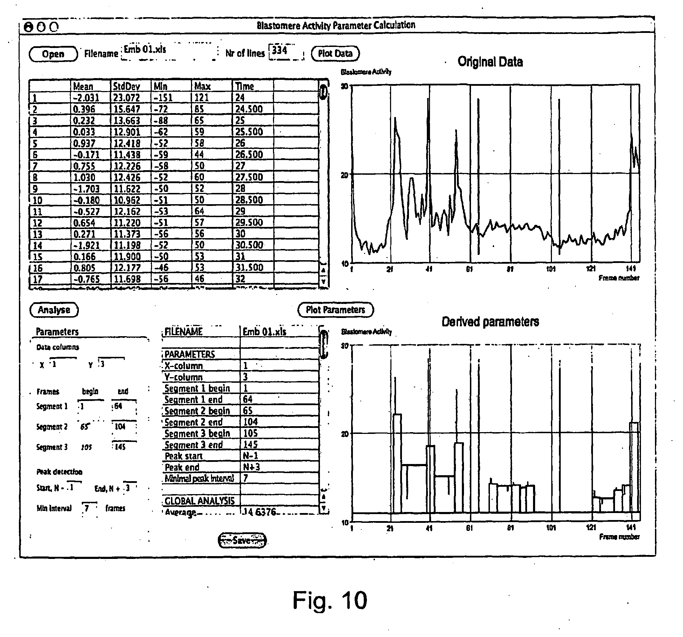

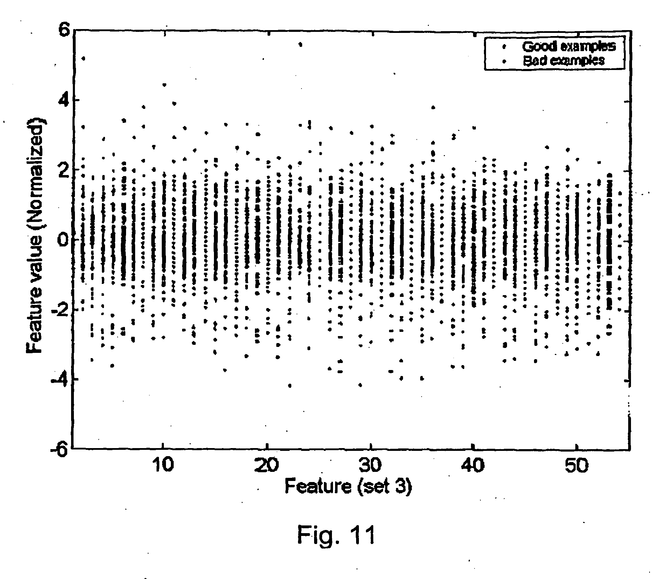

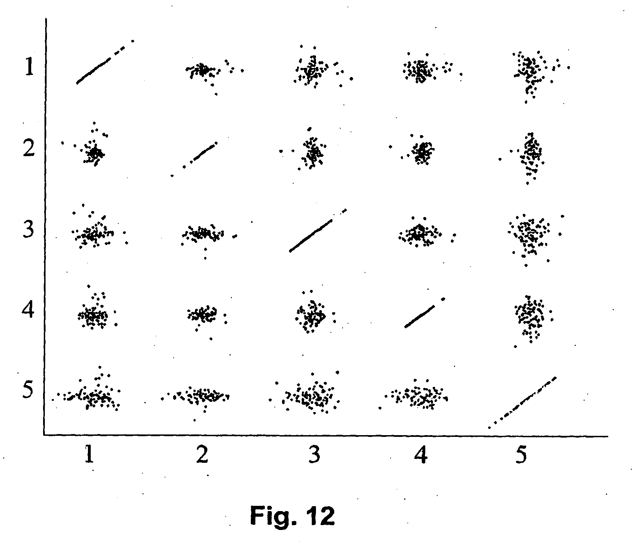

*Results.* A typical time series of blastomere activities consist of a few measurements every hour during incubation (e.g. approximately 150 data points for each embryo measured during the first 2 to 3 days which is the diagnostically interesting time window). Most statistical methods have difficulties with analysing data with such a high dimension. Thus, it is important to find robust methods for reducing the dimensions by extracting derived parameters. To achieve this, the blastomere activity was divided into three intervals: 0-32, 32-52 and 52-72 hours after image acquisition was started (Fig. 9). Within each of these intervals three peaks were found using the following method: The first peak was the highest blastomere activity. The second peak was the highest activity value that was at least 3.5h before or after the first peak. The third peak was the highest activity that was at least 3.5h from both the first and second peak. From each peak the following parameters were derived: the time, the peak value and the mean of the activity values from 0.5h before the peak to 1.5h after the peak. In addition, the valley between two peaks was described by the lowest value, the time of lowest value and the mean (see Fig. 10 for an example of the derived parameters). If the derived parameter values for different embryos are normalized to equal variance and mean value, it becomes apparent that aberrant values (i.e. too high or too low) are found for embryos that do not develop properly (bad embryos = blue dots in Fig. 11). Embryos that develop well (red dots) have a narrower range of values. Statistical models of embryo quality can be developed based on the above derived parameters. If each embryo has be evaluated according to the final development a number of different statistical methods exists for analysis the relation between the derived parameters and the final development. These methods includes: linear and non-linear models, Bayesians network, neural networks, hidden Markov models, nearest neighbours, principal component analysis and others. Fig. 12 below shows an example of a Principal Component Analysis (PCA) of the data.

The statistical model can be evaluated and/or extended as new data are generated. To facilitate this it is important to find a robust data structure and set of derived parameters.

Even very simple analysis of individual parameters such as parameter 39 = baseline value of blastomere activity in the third time segment (76 to 96 hrs after fertilization) can to some extend to sort out abnormal and non-viable embryos. Based on this single parameter it is thus possible to automatically select embryos of good quality with with 72 % accuracy.

## Example 6B

*Materials and methods.* Same as for Example 1

*Results.* A typical time series of blastomere activities consist of a few measurements every hour during incubation (e.g. approximately 150 data points for each embryo measured measured during the first 2 to 3 days which is the diagnostically interesting time window). Most statistical methods have difficulties with analysing data with such a high dimension. Thus, it is important to find robust methods for reducing the dimensions by extracting derived parameters. To achieve this, the blastomere activity was divided into three intervals: 0-32, 32-52 and 52-72 hours after image acquisition was started (Figure 9). The three time intervals was selected to reflect three developmental stages for bovine embryos. *Segment 1:* initial cell divisions from 1-cell to 8-cells. *Segment 2:* resting stage with relatively little activity and movements. It is believed the embryonic genome is activated at this stage. In some embryos the resting stage start at the 8-cell level, in others at the 16-cell stage, but in all developing embryos it is a prolonged period without cell divisions.

*Segment 3:* Resuming cell division an developing into a morula. It is often impossible to count individual blastomeres at this stage, but the time-lapse images reveal that cell division has resumed. Within each of the three time intervals reflecting the three developmental stages three peaks in blastomere activity were identified using the following method: The first peak was the highest blastomere activity. The second peak was the highest activity value that was at least 3.5h before or after the first peak. The third peak was the highest activity that was at least 3.5h from both the first and second peak.

From each peak the following parameters were derived: the time of occurrence, the peak value and the mean of the activity values from 0.5h before the peak to 1.5h after the peak. In addition, the valley between two peaks was described by the lowest value, the time of lowest value and the mean of the (see Figure 9 for an example of the derived parameters).

We thus get the following parameters for each of the three segments:

| 1 | Peak 1, value |
|---|---|
| 2 | Peak 1 time |
| 3 | Peak 1 mean |
| 4 | Valley 1, value |
| 5 | Valley 1 time |
| 6 | Valley 1 mean |
| 7 | Peak 2, value |
| 8 | Peak 2 time |
| 9 | Peak 2 mean |
| 10 | Valley 2, value |
| 11 | Valley 2 time |
| 12 | Valley 2 mean |
| 13 | Peak 3, value |
| 14 | Peak 3 time |
| 15 | Peak 3 mean |

In addition we calculate the average value and the standard deviation of blastomere activity in that segment:

| 16 | Average |
|---|---|
| 17 | StDev |

We also use some of the above parameters to describe the peak shape which reflects the duration or synchrony of the mayor cell division event. I sharp peak in blastomere activity (i.e. a fast synchronized cell division) is characterized by a low ratio of peak mean to peak value, whereas a higher ratio reflects a broader peak where the peak mean and peak values are more similar. Peak mean divided by peak value will always be < 1, with a value close to one indicating a broad peak and a value close to 0 a very sharp peak.

| 18 | (Peak 1, mean -Average) / (Peak 1, value - Average) = (P1=P16)/(P3-P16) |
|---|---|
| 19 | (Peak 2, mean - Average) / (Peak 2, value - Average) = (P7-P16)/(P9-P16) |
| 20 | (Peak 3, mean - Average) / (Peak 3, value - Average) = (P13-P16)/((P15-P16) |

Finally we calculate the ratio of the time between first and second peak and the ratio of time between the second and the third peak.

| 21 | (Peak 2, time - Peak 1, time) / (Peak 3, time - Peak 2, time) = (P8-P2)/(P14-P8) |
|---|---|

The parameter set of 21 parameters shown above is used for a fast analysis as it only include information that can be gained from the first segment i.e. 32 hours of incubation.

The small set contain important information that can me used to classify embryos in viable and not viable. However, if data for the following two time intervals is available then the analysis can be repeated for the two following segments. We do not calculate the ratios (i.e. shape characteristics and interval between peaks) for the following segments but only the peaks and valleys (i.e. 15 parameters per segment) Finally the global average value, the global StDev and the global Minimum and maximum are included in the full parameter set of 59 parameters shown below:

Glo Average
Glo StDev for BlastAct
Glo Minimum for BlastAct
Glo Maximum for BlastAct
SEG1 Average
SEG1 StDev
SEG1 Peak 1, value
SEG1 Peak 1 pos
SEG1 Peak 1 mean
SEG1 Valley 1, value
SEG1 Valley 1 pos
SEG1 Valley 1 mean
SEG1 Peak 2, value
SEG1 Peak 2 pos
SEG1 Peak 2 mean
SEG1 Valley 2, value
SEG1 Valley 2 pos
SEG1 Valley 2 mean
SEG1 Peak 3, value
SEG1 Peak 3 pos
SEG1 Peak 3 mean
SEG2 Average
SEG2 StDev
SEG2 Peak 1, value
SEG2 Peak 1 pos
SEG2 Peak 1 mean
SEG2 Valley 1, value
SEG2 Valley 1 pos
SEG2 Valley 1 mean
SEG2 Peak 2, value
SEG2 Peak 2 pos
SEG2 Peak 2 mean
SEG2 Valley 2, value
SEG2 Valley 2 pos
SEG2 Valley 2 mean
SEG2 Peak 3, value SEG2 Peak 3 pos
SEG2 Peak 3 mean
SEG3 Average
SEG3 StDev
SEG3 Peak 1, value
SEG3 Peak 1 pos
SEG3 Peak 1 mean
SEG3 Valley 1, value
SEG3 Valley 1 pos
SEG3 Valley 1, mean
SEG3 Peak 2, value
SEG3 Peak 2 pos
SEG3 Peak 2 mean
SEG3 Valley 2, value
SEG3 Valley 2 pos
SEG3 Valley 2 mean
SEG3 Peak 3, value
SEG3 Peak 3 pos
SEG3 Peak 3 mean
SEG1 ratio peak1
SEG1 ratio peak2

SEG1 ratio peak3
SEG1 ratio val1 val2

If the derived parameter values for different embryos are normalized to equal variance and mean value, it becomes apparent that aberrant values (i.e. too high or too low) are found for embryos that do not develop properly (bad embryos = blue dots in figure 11). The parameters in the figure are in the same order as the above but the four ratio parameters at the end are omitted. Embryos that develop well (red dots) have a narrower range of values.

Statistical models of embryo quality can be developed based on the above derived parameters. If each embryo has be evaluated according to the final development a number of different statistical methods exists for analysis the relation between the derived parameters and the final development. These methods includes but are not limited to: linear and non-linear models, Bayesians network, neural networks, hidden Markov models, nearest neighbours, principal component analysis and others. Figure 11 below shows an example of a Principal Component Analysis (PCA) of the data.

An example of the use of a linear model is shown in Example 7.

The statistical model can be evaluated and/or extended as new data are generated. To facilitate this it is important to find a robust data structure and set of derived parameters.

Even very simple analysis of individual parameters such as parameter 39 = baseline value of blastomere activity in the third time segment (76 to 96 hrs after fertilization) can to some extend to sort out abnormal and non-viable embryos. Based on this single parameter it is thus possible to automatically select embryos of good quality with with 72 % accuracy.

**Example 7.** Comparison of selection of embryos based on automated detection or embryologist detection

*Design.* 95 bovine embryos were placed in a time-lapse microscope under constant temperature, humidity and $CO_2$ for seven days. Images were acquired twice per hour from 24 hours to 96 hours after fertilization. The ability of the image-analysis procedure to correctly identify the 38 embryos that subsequently (i.e. after 7 days) developed to expanded blastocysts was evaluated and compared to the quality assessments by a trained embryologist based on the same 145 images for each embryo.

*Material & Methods.* Bovine immature cumulus-oocyte complexes were aspirated from slaughterhouse-derived ovaries, matured for 24h before fertilization for 22h. Cumulus cells were then removed and presumptive zygotes were transferred and cultured in synthetic oviduct fluid medium. Time-lapse images were acquired inside an incubator box fitted onto an inverted Nikon microscope stage mounted with a sensitive video camera.

*Results.* The fully automated image analysis procedure generated a quantitative measure of cell blastomere activity based on the observed movement between consecutive images in the time-lapse series. The correlation between blastomere activity and cell division was confirmed by comparing automated and manual analysis of the time-lapse image series. Pronounced peaks in blastomere activity were found to be associated with cell-divisions. The exact onset and duration of cell-divisions could be quantified based on position, shape and size of the recorded peaks. The blastomere activity pattern of a given embryo could thus be reduced to a set of key parameters corresponding to peak height, position and width for prominent peaks as well as similar parameters describing the blastomere activity level between peaks. A total of 55 parameters for each embryo was used in a simple linear model to classify the embryo as "viable" or "non-viable". The model was trained on a subset of the observed embryo patterns and evaluated on a different independent subset. The same time-lapse series of images was evaluated by a skilled embryologist attempting to predict whether the embryo would develop to an expanded blastocyst or not.

Though the model was only a simple linear model with limited accuracy it was noted that the fully automated analysis was better at predicting which embryos would develop to expanded blastocysts (Error rate: 20%, 24 out of 94), than the trained embryologist (Error rate 26%, 19 out of 95), Moreover the automated analysis also had fewer false positives (13 of 45 = 29%, as opposed to the manual analysis which had (23 of 60, 38%). False positives are embryos that are believed to have a high viability but nevertheless cease development and never reached the expanded blastocyst stage within the 7-day observation period. Transfer of such embryos are unlikely to result in pregnancy.

**Bovine embryo Experiment segment 1 - 3**

Images acquired every 30 min from 24 hrs to 96 hrs after fertilization
Outcome evaluated after 7 days = End point
(N=94, blastocystrate=40%)

| Outcome | Manual Evaluation | | Image analysis | |
|---|---|---|---|---|
| | **Good** | **Bad** | **Good** | **Bad** |
| **Expanding blastocysts** | 37 | 1 | 32 | 6 |

(continued)

| Outcome | Manual Evaluation | | Image analysis | |
|---|---|---|---|---|
| **Arrested development** | 23 | 33 | 13 | 44 |
| Incorrect classified | **26%** | | **20%** | |
| False positives & negatives | **38%** | 3% | **29%** | 12% |

**References**

***Articles***

Beliën JAM, Baak JPA, Van Diest PJ and Van Ginkel AHM (1997) Counting mitoses by image processing in feulgen stained breast cancer sections: The influence of resolution. Cytometry 28: 135-140

Bhattacharya S and Templeton A (2004). What is the most relevant standard of success in assisted reproduction? Redefining success in the context of elective single embryo transfer: evidence, intuition and financial reality. Human reproduction page 1 - 4

Bos-Mikich A, Mattos ALG and Ferrari AN (2001) Early cleavage of human embryos: an effective method for predicting successful IVF/ICSI outcome. Hum Reprod 16,2658-2661.

Curl CL, Harris T, Harris PJ, Allman BE, Bellair CJ, Stewart AG and Delbridge LMD (2004) Quantitative phase microscopy: a new tool for measurement of cell culture growth and confluency in situ. Pflugers Arch - Eur J Physiol 448: 462-468

Eccles BA and Klevecz RR (1986) Automatic digital image analysis for identification of mitotic cells in synchronous mammalian cell cultures. Pubmed, anal quant cytol histol 8:138-47

Fenwick J, Platteau P, Mucdoch AP and Herbert M (2002) Time from insemination to first cleavage predicts developmental competence of human preimplantation embryos in vitro. Hum reprod 17,407-412

Grisart B, Massip a and Dessy F (1994) Cinematographic analysis of bovine embryo development in serum-free oviduct-conditioned medium. Pubmed, J. Reprod fertile 101(2): 257-64

Haney S.M, Thompson PM, Cloughesy TF, Alger JR and Toga AW (2001) Tracking tumor growth rates in Patients with Malignant gliomas: A test of two algorithms. AJNR An J Neuroradiol 22:73-82

Christina Hnida (2004) Computer-assisted, multilevel, morphometric analysis of biomarkers of embryonic quality. PhD thesis, University of Copenhagen

Holm P, Booth PJ and Callesen H (2002) Kinetics of early in vitro development of bovine in vivo-and in vitro-derived zygotes produced and/or cultured in chemically defined or serum-containing media. Reproduction 123: 553-565

Holm P, Booth PJ and Callesen H (2003) Developmental kinetics of bovine nuclear transfer and parthenogenetic embryos. Cloning and stem cell Vol 5 number 2

Holm P, Shukri NN, Vajta G, Booth P, Bendixen C and Callesen H (1998) Developmental kinetics of the first cell cycles of bovine in vitro produced embryos in relation to their in vitro viability and sex. Theriogenology 50: 1285-1299

Lundin K, Bergh C and Harderson T (2001) Early embryo cleavage is a strong indicator of embryo quality in human IVF. Hum Reprod 16,2652-2657

Majerus V, Lequarre AS, Ferguson EM, Kaidi S, Massip A, Dessy F and Donnay I (2000) Characterization of embryos derived from calf oocytes: Kinetics of cleavage, cell allocation to Inner cell mass, and trophectoderm and lipid metabolism. Molecular reproduction and development 57: 346-352

Motosugi N, Bauer T, Polanski Zbigniew, Solter D and Hiiragi T (2005) Polarity of the mouse embryo is established at blastocyst and is not prepatterned. Genes & development 19: 1081-1092

Oberholzer M, Ostrelcher M, Christen H and Bruhlmann M (1996) Methods in quantitative Image analysis. Pubmed, histochem cell boil 105: 333-55

Petersen CG, Mauri AL, Ferreira R, Baruffi RLR and Franco Jr JG (2001). Embryo selection by the first cleavage parameter between 25 and 27 hours after ICSI. J Assist Reprod Genet 18,209-212

Sakkas D, Percival G, D'arcy Y, Sharif K and Afnan M (2001) Assessment of early cleaving in vitro fertilized human embryos at the 2-cell stage before transfer improves embryo selection. Fertil Steril 76,1150-1156

Sakkas D, Shoukir Y, Chardonnens D, Bianchi PG and Campana A (1998) Early cleavage of human embryos to the two-cell stage after Intracytoplasmic sperm injection as an indicator of embryo viability. Hum Reprod 13,182-187

Salumets A, Hydén-Granskog C, Suikkari A-M, Tiitinen A and Tuuri T (2002) The predictive value of pronuclear morphology of zygotes in the assessment of human embryo quality. Hum Reprod 16,2177-2181

Shoukir Y, Campana A, Farley T and Sakkas D (1997) Early cleavage of in-vitro fertilized embryos to the 2-cel/ stage: a novel indicator of embryo quality and viability. Hum Reprod 12,1531-1536

Squirrell, J M et al (1999) Long term two-photon fluorescence imaging of mammalian embryos without compromising viability, Nature Biotechnology, vol. 11, no. 17, 763-767

Squirrell, J M et al (2003) Imaging mitochondrial organization in living primate oocytes and embryos using multiphoton microscopy' Microscopy and micranalysis, vol. 9, no. 3, 190-201

Tokura, T et al. (1993) Sequential observation of mitochondrial distribution in mouse oocytes and embryos, Journal of assisted reproduction and genetics, vol. 10, no. 6, 417-426

Vayena E, Rowe PJ and Griffin PD (2001) Current practices and controversies in assisted reproduction: Report of a meeting on "Medical, Ethical and Social aspects of assisted reproduction" held at WHO headquarters in Geneva, Switzerland.

Windt M-L, Krueger TF, Coetzee K and Lombard CJ (2004) Comparative analysis of pregnancy rates after the transfer of early dividing embryos versus slower dividing embryos. Hum Reprod Vol 19 No 5 pp 1155-1162

***Books***

John C Russ (2002) The Image Processing Handbook, CRC press, 4'th Edition ISBN: 084931142X

***Patents***

Bongiovanni Kevin Paul, Audi Paul Patrick, Fortin Christophers, McPhillips Kenneth (24/02/2005) *Automatic target detection and motion analysis form image data.* US2005041102

Cecchi Michael D, Mezezi Monica (24/07/2003) *Biological specimen-culturing system and method with onboard specimen development sensors.* US2003138942

Iwasaki Masahiro, Imagawa Taro (28/04/2005) *Monitoring device.* WO2005039181

Myers James Carrol (25/06/2004) *A method of searching recorded digital video for areas of activity* MXA03003268, U36434320 (E1)

Klevecz Robert R., Eccles Beverly A. (09/02/1988) *Method and apparatus for automatic digital image analysis.* US4724543

Tago Akira, Tsujii Osamu (18/05/2005) *Radiographic image processing method and apparatus.* EP1531422.

**Claims**

1. A method for determining embryo quality comprising monitoring the embryo for a time period before the embryo undergoes compaction, said time period having a length sufficient to comprise at least one cell division period and at least a part of an inter-division period, and determining the extent of cellular movement during the at least part of an inter-division period thereby obtaining an embryo quality measure.

2. The method according to claim 1, wherein the embryo is monitored for a time period comprising at least two cell division periods.

3. The method according to claim 1, wherein the embryo is monitored for a time period comprising at least three cell division periods.

4. The method according to any of the preceding claims, wherein the length of the at least one cell division period is determined.

5. The method according to any of the preceding claims, wherein the duration of an inter-division period is determined.

6. The method according to any of the preceding claims, wherein the extent and/or spatial distribution of cellular or organelle movement during cell division periods is determined.

7. The method according to any of the preceding claims, wherein the length of each cell division period is determined.

8. The method according to any of the preceding claims, wherein the length of each inter-division period is determined.

9. The method according to any of the preceding claims, wherein the period of cellular movement is determined in at least two inter-division periods.

10. The method according to any of the preceding claims, wherein the extent of cellular movement is determined in at least two inter-division periods.

11. The method according to claim 9 or 10, wherein the at least two inter-division periods are subsequent inter-division periods.

12. The method according to any of the preceding claims, wherein determination of the length of the at least one cell division period and determination of the period of cellular movement during the inter-division periods are performed.

13. A method for selecting an embryo suitable for transplantation, said method comprising monitoring the embryo as defined in any of the claims 1-12 obtaining an embryo quality measure, and selecting the embryo having the highest embryo quality measure.

**Patentansprüche**

1. Ein Verfahren zur Bestimmung der Qualität von Embryonen umfassend die Überwachung des Embryos für eine Zeitspanne bevor der Embryo kompaktiert, wobei die besagte Zeitspanne eine ausreichende Länge hat, um zumindest eine Zellteilungsperiode und zumindest einen Teil einer bereichsübergreifenden Periode zur umfassen, und die Bestimmung des Gehaltes an zellulärer Bewegung während des zumindest einen Teils einer bereichsübergreifenden Periode, wodurch ein Embryoqualitätsmaß erhalten wird.

2. Ein Verfahren nach Anspruch 1, wobei der Embryo für eine Zeitspanne überwacht wird, die wenigstens zwei Zellteilungsperioden umfasst.

3. Ein Verfahren nach Anspruch 1, wobei der Embryo für eine Zeitspanne überwacht wird, die wenigstens drei Zellteilungsperioden umfasst.

4. Ein Verfahren nach einem der vorherigen Ansprüche, wobei die Dauer der zumindest einen Zellteilungsperiode bestimmt wird.

5. Ein Verfahren nach einem der vorherigen Ansprüche, wobei die Dauer einer bereichsübergreifenden Periode bestimmt wird.

6. Ein Verfahren nach einem der vorherigen Ansprüche, wobei der Gehalt und/oder die räumliche Verteilung von zellulärer oder organeller Bewegung während der Zellteilungsperiode bestimmt wird.

7. Ein Verfahren nach einem der vorherigen Ansprüche, wobei die Dauer jeder Zellteilungsperiode bestimmt wird.

8. Ein Verfahren nach einem der vorherigen Ansprüche, wobei die Dauer jeder bereichübergreifenden Periode bestimmt wird.

9. Ein Verfahren nach einem der vorherigen Ansprüche, wobei in wenigstens zwei bereichsübergreifenden Perioden die Periode zellulärer Bewegung bestimmt wird.

10. Ein Verfahren nach einem der vorherigen Ansprüche, wobei in wenigstens zwei bereichsübergreifenden Perioden der Gehalt an zellulärer Bewegung bestimmt wird.

11. Ein Verfahren nach Anspruch 9 oder 10, wobei die wenigstens zwei bereichsübergreifenden Perioden aufeinanderfolgende bereichsübergreifende Perioden sind.

12. Ein Verfahren nach einem der vorherigen Ansprüche, wobei die Bestimmung der Länge der wenigstens einen Zellteilungsperiode und die Bestimmung der Periode zellulärer Bewegung während der bereichsübergreifenden Periode bestimmt wird.

13. Ein Verfahren zur Selektion eines Embryos, der für die Transplantation geeignet ist, wobei besagte Methode die Beobachtung von Embryonen umfasst, wie in einem der Ansprüche 1 bis 12 definiert, zum Erhalt eines Embryoqualitätsmaßes, und die Auswahl des Embryos mit dem höchsten Embryoqualitätsmaß.

**Revendications**

1. Procédé de détermination de la qualité d'un embryon comprenant la surveillance de l'embryon pendant une durée avant que l'embryon subisse un compactage, ladite durée avant une durée suffisante pour comprendre au moins une période de division cellulaire et au moins une partie d'une période entre les divisions, et la détermination de l'étendue du mouvement des cellules pendant la ou les parties d'une période entre les divisions donnant ainsi une mesure de la qualité d'un embryon.

2. Procédé selon la revendication 1, dans lequel l'embryon est surveillé pendant une durée comprenant au moins deux périodes de division cellulaire.

3. Procédé selon la revendication 1, dans lequel l'embryon est surveillé pendant une durée comprenant au moins trois périodes de division cellulaire.

4. Procédé selon l'une quelconque des revendications précédentes, dans lequel la durée de la ou des périodes de division cellulaire est déterminée.

5. Procédé selon l'une quelconque des revendications précédentes, dans lequel la durée d'une période entre les divisions est déterminée.

6. Procédé selon l'une quelconque des revendications précédentes, dans lequel l'étendue et/ou la distribution spatiale du mouvement des cellules ou des organelles pendant les périodes de division cellulaire est déterminée.

**7.** Procédé selon l'une quelconque des revendications précédentes, dans lequel la durée de chaque période de division cellulaire est déterminée.

**8.** Procédé selon l'une quelconque des revendications précédentes, dans lequel la durée de chaque période entre les divisions est déterminée.

**9.** Procédé selon l'une quelconque des revendications précédentes, dans lequel la période de mouvement des cellules est déterminée dans au moins deux périodes entre les divisions.

**10.** Procédé selon l'une quelconque des revendications précédentes, dans lequel l'étendue du mouvement des cellules est déterminée dans au moins deux périodes entre les divisions.

**11.** Procédé selon la revendications ou 10, dans lequel les au moins deux périodes entre les divisions sont des périodes entre les divisions consécutives.

**12.** Procédé selon l'une quelconque des revendications précédentes, dans lequel la détermination de la durée de la ou des périodes de division cellulaire et la détermination de la période de mouvement des cellules pendant les périodes entre les divisions sont réalisées.

**13.** Procédé de sélection d'un embryon approprié pour une greffe, ledit procédé comprenant la surveillance de l'embryon tel que défini selon l'une quelconque des revendications 1 à 12, permettant d'obtenir une mesure de la qualité d'un embryon, et de choisir l'embryon ayant la mesure de qualité d'embryon la plus élevée.

Fig. 2

Fig. 3

Fig. 4

Good     Bad

Fig. 5

Fig. 6

| | |
|---|---|
| —— 04-StDev | |
| —— 05-StDev | |
| —— 07-StDev | |
| —— 09-StDev | |
| —— 11-StDev | |
| —— 12-StDev | |
| —— 13-StDev | |
| —— 14-StDev | |
| —— 15-StDev | |
| —— 16-StDev | |
| —— 18-StDev | |
| —— 27-StDev | |
| —— 31-StDev | |
| —— 33-StDev | |
| —— 34-StDev | |
| —— 35-StDev | |
| —— 36-StDev | |
| —— 38-StDev | |
| —— Part 1 | |
| —— Part 2 | |
| —— Part 3 | |

Time (t)

**Fig. 7**

Automatic (frame)

60.0

40.0

20.0

0.0

6/9

◇ 1 to 2
■ 2 to 4
4 to 8

0.0    20.0    40.0    60.0

Manual (frame)

| | Manual analysis | | | | Automated analysi | | |
|---|---|---|---|---|---|---|---|
| | 1 to 2 | 2 to 4 | 4 to 8 | | 1 to 2 | 2 to 4 | 4 to 8 |
| 1 | 23.5 | 40.5 | 54.5 | | 23.5 | 40.5 | 54.5 |
| 2 | 29.0 | 46.5 | 66.5 | | 29.5 | 46.5 | |
| 3 | 16.5 | 32.5 | | | 16.5 | 32.5 | |
| 4 | 10.5 | 26.5 | 36.5 | | 10.5 | 24.5 | 37.5 |
| 5 | 12.5 | 27.5 | 39.5 | | 12.5 | | 39.5 |
| 6 | 18.5 | 35.5 | 50.0 | | 18.5 | 35.5 | |
| 7 | 16.5 | 32.5 | 46.5 | | 16.5 | 32.5 | 47.5 |
| 8 | 18.5 | 36.5 | 50.0 | | 18.5 | 36.5 | 50.5 |
| 9 | 1.5 | 15.5 | 33.5 | | | 15.5 | 32.5 |
| 10 | 17.5 | 32.5 | 48.5 | | 17.5 | 32.5 | |
| 11 | 22.5 | 37.5 | 51.0 | | 22.5 | 37.5 | 50.5 |
| 12 | 16.5 | 30.5 | 43.0 | | 15.5 | 30.5 | 42.5 |
| 13 | 17.0 | 33.5 | 49.0 | | 17.5 | 33.5 | 48.5 |

Fig. 8

Cell divisions

Fig. 9

Fig. 10

Fig. 11

**Fig. 12**

Fig. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007042044 A
- WO 2004056265 A
- US 2005041102 A
- US 2003138942 A
- WO 2005039181 A
- US 4724543 A
- EP 1531422 A

### Non-patent literature cited in the description

- **Beliën JAM ; Baak JPA ; Van Diest PJ ; Van Ginkel AHM.** Counting mitoses by image processing in feulgen stained breast cancer sections: The influence of resolution. *Cytometry,* 1997, vol. 28, 135-140
- **Bhattacharya S ; Templeton A.** What is the most relevant standard of success in assisted reproduction? Redefining success in the context of elective single embryo transfer: evidence, intuition and financial reality. *Human reproduction,* 1-4
- **Bos-Mikich A ; Mattos ALG ; Ferrari AN.** Early cleavage of human embryos: an effective method for predicting successful IVF/ICSI outcome. *Hum Reprod,* 2001, vol. 16, 2658-2661
- **Curl CL ; Harris T ; Harris PJ ; Allman BE ; Bellair CJ ; Stewart AG ; Delbridge LMD.** Quantitative phase microscopy: a new tool for measurement of cell culture growth and confluency in situ. *Pflugers Arch - Eur J Physiol,* 2004, vol. 448, 462-468
- **Eccles BA ; Klevecz RR.** Automatic digital image analysis for identification of mitotic cells in synchronous mammalian cell cultures. *Pubmed, anal quant cytol histol,* 1986, vol. 8, 138-47
- **Fenwick J ; Platteau P ; Mucdoch AP ; Herbert M.** Time from insemination to first cleavage predicts developmental competence of human preimplantation embryos in vitro. *Hum reprod,* 2002, vol. 17, 407-412
- **Grisart B ; Massip a ; Dessy F.** Cinematographic analysis of bovine embryo development in serum-free oviduct-conditioned medium. *Pubmed, J. Reprod fertile,* 1994, vol. 101 (2), 257-64
- **Haney S.M ; Thompson PM ; Cloughesy TF ; Alger JR ; Toga AW.** Tracking tumor growth rates in Patients with Malignant gliomas: A test of two algorithms. *AJNR An J Neuroradiol,* 2001, vol. 22, 73-82
- Computer-assisted, multilevel, morphometric analysis of biomarkers of embryonic quality. **Christina Hnida.** PhD thesis. University of Copenhagen, 2004
- **Holm P ; Booth PJ ; Callesen H.** Kinetics of early in vitro development of bovine in vivo-and in vitro-derived zygotes produced and/or cultured in chemically defined or serum-containing media. *Reproduction,* 2002, vol. 123, 553-565
- **Holm P ; Booth PJ ; Callesen H.** Developmental kinetics of bovine nuclear transfer and parthenogenetic embryos. *Cloning and stem cell,* 2003, vol. 5 (2
- **Holm P ; Shukri NN ; Vajta G ; Booth P ; Bendixen C ; Callesen H.** Developmental kinetics of the first cell cycles of bovine in vitro produced embryos in relation to their in vitro viability and sex. *Theriogenology,* 1998, vol. 50, 1285-1299
- **Lundin K ; Bergh C ; Harderson T.** Early embryo cleavage is a strong indicator of embryo quality in human IVF. *Hum Reprod,* 2001, vol. 16, 2652-2657
- **Majerus V ; Lequarre AS ; Ferguson EM ; Kaidi S ; Massip A ; Dessy F ; Donnay I.** Characterization of embryos derived from calf oocytes: Kinetics of cleavage, cell allocation to Inner cell mass, and trophectoderm and lipid metabolism. *Molecular reproduction and development,* 2000, vol. 57, 346-352
- **Motosugi N ; Bauer T ; Polanski Zbigniew ; Solter D ; Hiiragi T.** Polarity of the mouse embryo is established at blastocyst and is not prepatterned. *Genes & development,* 2005, vol. 19, 1081-1092
- **Oberholzer M ; Ostrelcher M ; Christen H ; Bruhlmann M.** Methods in quantitative Image analysis. *Pubmed, histochem cell boil,* 1996, vol. 105, 333-55
- **Petersen CG ; Mauri AL ; Ferreira R ; Baruffi RLR ; Franco Jr JG.** Embryo selection by the first cleavage parameter between 25 and 27 hours after ICSI. *J Assist Reprod Genet,* 2001, vol. 18, 209-212

- **Sakkas D ; Percival G ; D'arcy Y ; Sharif K ; Afnan M.** Assessment of early cleaving in vitro fertilized human embryos at the 2-cell stage before transfer improves embryo selection. *Fertil Steril,* 2001, vol. 76, 1150-1156
- **Sakkas D ; Shoukir Y ; Chardonnens D ; Bianchi PG ; Campana A.** Early cleavage of human embryos to the two-cell stage after Intracytoplasmic sperm injection as an indicator of embryo viability. *Hum Reprod,* 1998, vol. 13, 182-187
- **Salumets A ; Hydén-Granskog C ; Suikkari A-M ; Tiitinen A ; Tuuri T.** The predictive value of pronuclear morphology of zygotes in the assessment of human embryo quality. *Hum Reprod,* 2002, vol. 16, 2177-2181
- **Shoukir Y ; Campana A ; Farley T ; Sakkas D.** Early cleavage of in-vitro fertilized embryos to the 2-cel/ stage: a novel indicator of embryo quality and viability. *Hum Reprod,* 1997, vol. 12, 1531-1536
- **Squirrell, J M et al.** Long term two-photon fluorescence imaging of mammalian embryos without compromising viability. *Nature Biotechnology,* 1999, vol. 11 (17), 763-767
- **Squirrell, J M et al.** Imaging mitochondrial organization in living primate oocytes and embryos using multiphoton microscopy'. *Microscopy and micranalysis,* 2003, vol. 9 (3), 190-201
- **Tokura, T et al.** Sequential observation of mitochondrial distribution in mouse oocytes and embryos. *Journal of assisted reproduction and genetics,* 1993, vol. 10 (6), 417-426
- **Vayena E ; Rowe PJ ; Griffin PD.** Current practices and controversies in assisted reproduction: Report of a meeting. *Medical, Ethical and Social aspects of assisted reproduction,* 2001
- **Windt M-L ; Krueger TF ; Coetzee K ; Lombard CJ.** Comparative analysis of pregnancy rates after the transfer of early dividing embryos versus slower dividing embryos. *Hum Reprod,* 2004, vol. 19 (5), 1155-1162
- **John C Russ.** The Image Processing Handbook. CRC press, 2002